# EUROPEAN PATENT APPLICATION

(11) **EP 1 468 997 A2**
(43) Date of publication of application: **20.10.2004**
(21) Application number: 04076138.9
(22) Date of filing: 13.04.2004
(51) Int. Cl.: C07D 417/12, A61K 31/427, A61P 43/00

(54) **Polymorphous forms of rosiglitazone maleate**

(30) Priority: 18.04.2003 IT mi20030820; 21.05.2003 US 472756 P
(71) Applicant: CHEMI S.p.A., 20092 Cinisello Balsamo (Milano) (IT)
(72) Inventor: Turchetta, Stefano, 00139 Roma (IT); Massardo, Pietro, 00154 Roma (IT); Aromatario, Valentina, 00177 Roma (IT)
(74) Representative: Pistolesi, Roberto

(57) **Abstract**

Three new polymorphous crystalline forms of rosiglitazone maleate, termed respectively form I, II and III and the methods for selectively obtaining each form are described and characterized. Rosiglitazone maleate may be obtained in the form of the single polymorph I by blending an approximately equimolar mixture of rosiglitazone base and maleic acid in a series of solvents and mixtures thereof which comprises isopropanol, acetone, ethyl acetate, isopropyl acetate, THF, followed by cooling of the mixture to ambient temperature; the form II may on the other hand be obtained by means of treatment of the approximately equimolar mixture of rosiglitazone base and maleic acid in water under reflux, followed by cooling of the mixture to ambient temperature; the polymorph III may be obtained by treating a mixture of rosiglitazone base with a double molar quantity of maleic acid in ethanolic solvents.

## Description

### FIELD OF THE INVENTION

The present invention relates to the synthesis and characterization of three polymorphous forms of rosiglitazone maleate.

### STATE OF THE ART

Rosiglitazone is a molecule of thiazolidinedione structure which forms part of the class of antidiabetics. Its structure formula is given below.

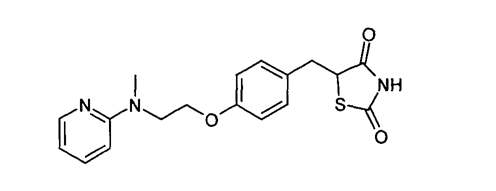

US 5,002,953 describes for the first time the compound and its use as an antihyperglycaemic. In that patent all its pharmaceutically acceptable salts are also claimed.

US 5,741,803 instead specifically describes the maleate of rosiglitazone, shown below, stating that among the possible salts, the maleate exhibits particularly favourable characteristics of stability and solubility in water.

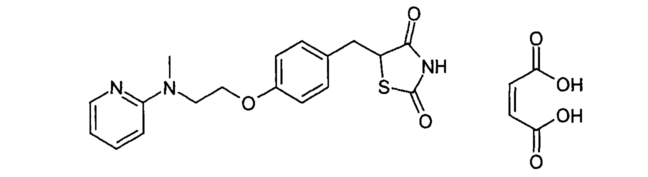

In that patent, two examples of the preparation of the salt in question are given. In the first example the compound is prepared by hot dissolution of the rosiglitazone base mixed with maleic acid, and slow precipitation of the salt derived therefrom. After treatment of the suspension at 0-5°C for several hours, a product is isolated which, when dried under vacuum at 50°C provides a product having a melting point (m.p.) of 120-121°C. The ¹H-NMR of the product is provided in which a wide band between 2 and 5 ppm is found which the applicant attributes to the residual water contained in the solvent (not otherwise specified). In the second example the maleate of rosiglitazone is treated, in ethanol, with an equivalent of maleic acid, while hot, until dissolution of the solid is obtained, the mixture is decoloured with carbon and the product is precipitated by cooling to 0-5°C, then the product is filtered and desiccated, having at the end of the treatments a m.p. of 119-119.5°C.

US 6,515,132 relates to a method for the synthesis of rosiglitazone maleate, in which the step of formation of the maleate of rosiglitazone is carried out in acetone.

Polymorphic forms of rosiglitazone maleate are disclosed in WO0064892, WO0064893, WO0064896 and WO0226737 whereas WO9931093 WO9931094 and WO9931095 describe the preparation of hydrates of rosiglitazone maleate.

### DESCRIPTION OF THE INVENTION

It is known in fact that many organic compounds and their salts may exist in the form of a plurality of different crystalline structures, which exhibit different physical properties and may exhibit differences also from the biological point of view.

In the course of experiments on crystallization of the maleate of rosiglitazone it was surprisingly found that this salt, under specific conditions, crystallizes in three different polymorphic crystalline pure forms, that have not been described before.

Obtaining pure crystalline forms is extremely useful, both because through these a precise characterization of the chemical-physical properties is possible, and because these characteristics may prove more favourable from a pharmacological point of view.

The subject of the present patent application are therefore three new polymorphous forms of rosiglitazone maleate, and also the methods necessary for the crystallization of these polymorphic forms.

### DETAILED DESCRIPTION OF THE INVENTION

Tests on the synthesis of rosiglitazone maleate carried out starting from equimolar amounts of rosiglitazone base and maleic acid surprisingly led to the identification and characterization of two polymorphous crystalline forms of the aforesaid salt. Moreover by crystallizing mixtures of rosiglitazone base and double equimolar quantities of maleic acid a third polymorph of rosiglitazone maleate is obtained.

In particular, it was found that the maleate of rosiglitazone exists in three polymorphous crystalline modifications, which may be easily distinguished both by means of DSC, and IR, and also X-ray diffraction.

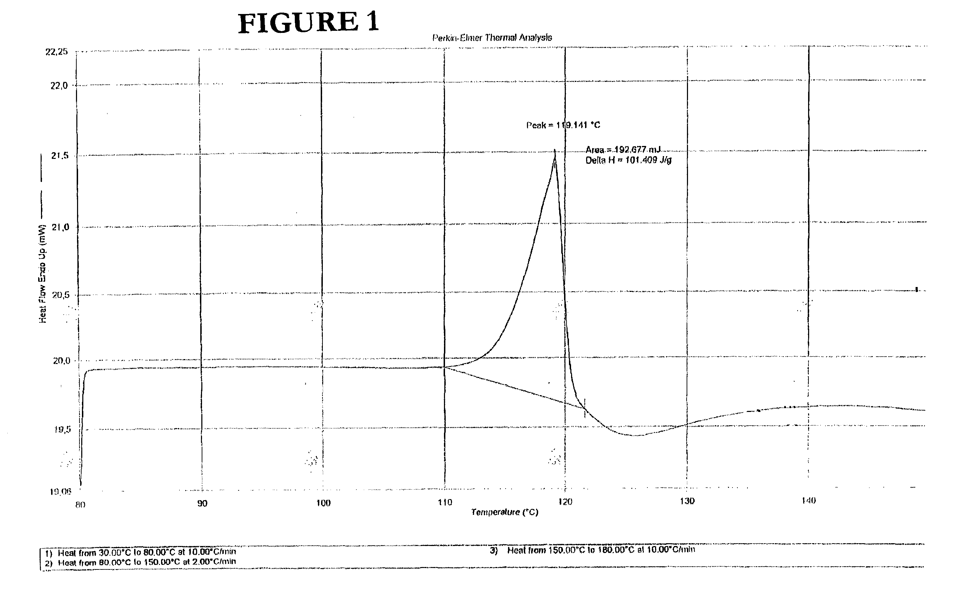

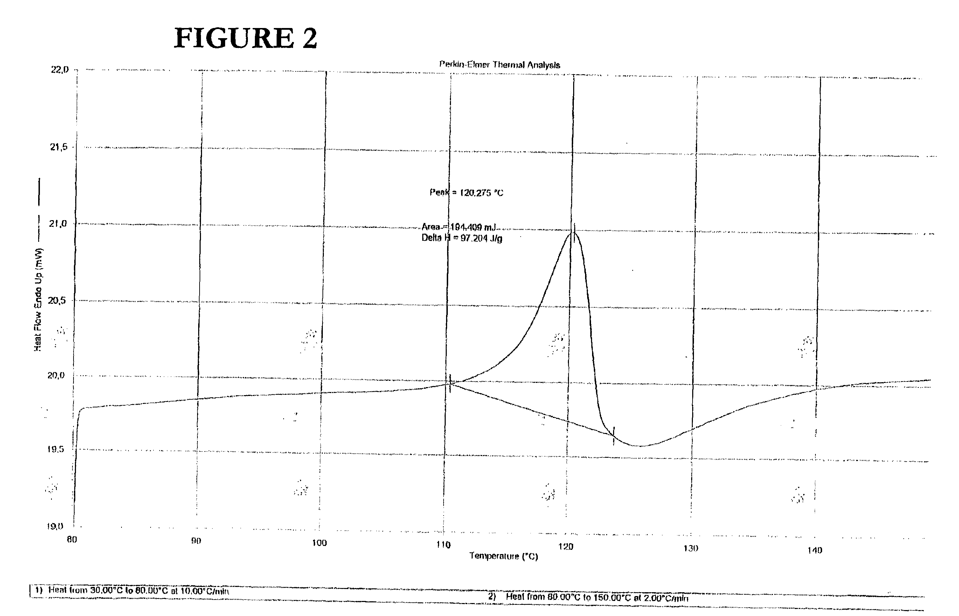

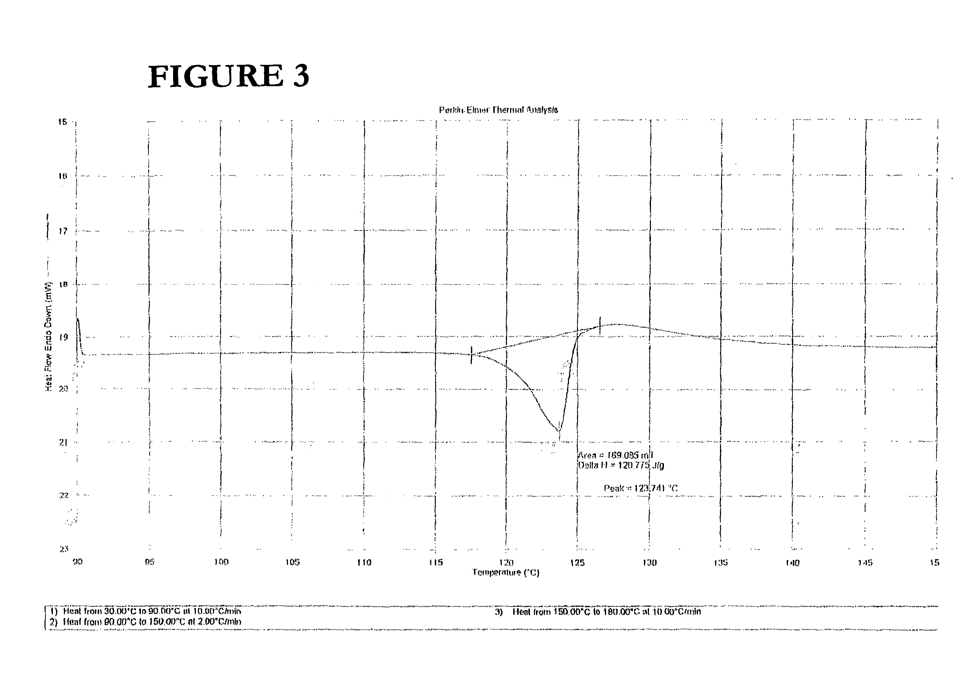

Rosiglitazone maleate exists in a polymorphous form I, which with the DSC exhibits an endothermic peak with maximum at 119°C (Figure 1), in a polymorphous form II, which with the DSC exhibits an endothermic peak with maximum at 121°C (Figure 2), and in a polymorphous form III which with the DSC exhibits an endothermic peak at 124°C (Figure 3) The DSCs were carried out with a Perkin Elmer DSC7 Differential Scanning Calorimeter.

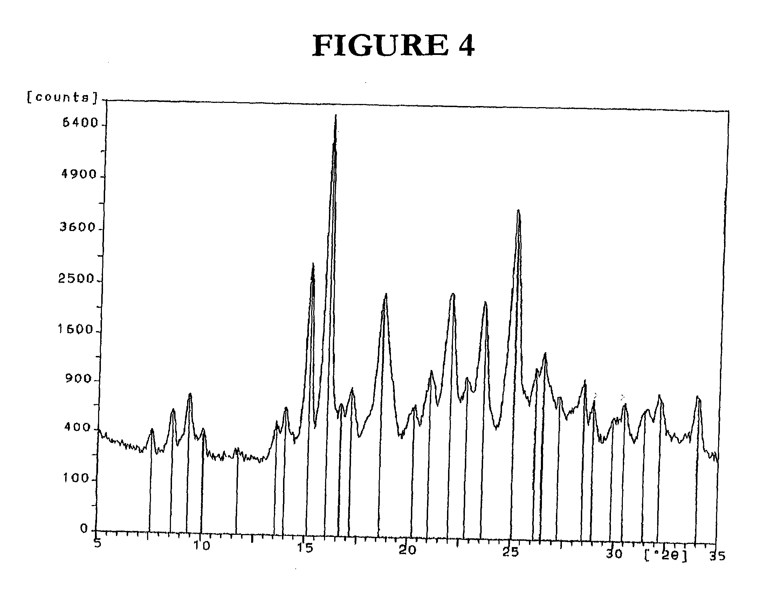

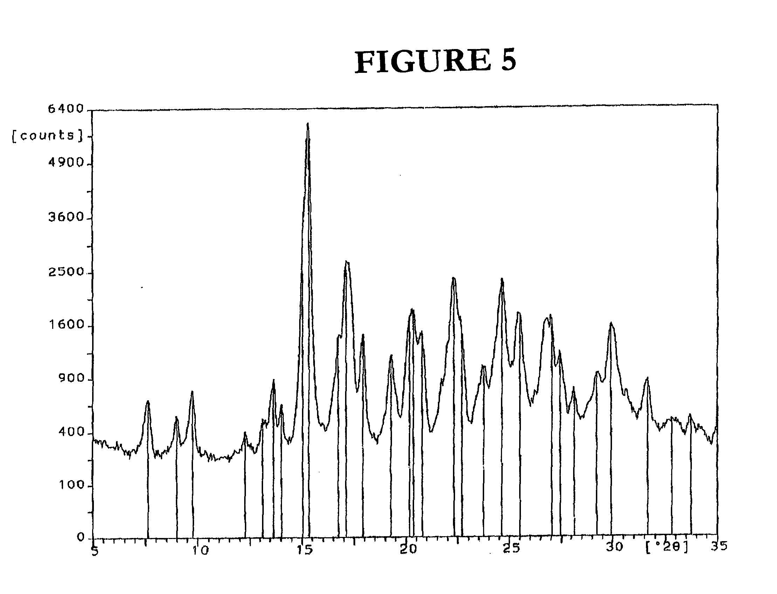

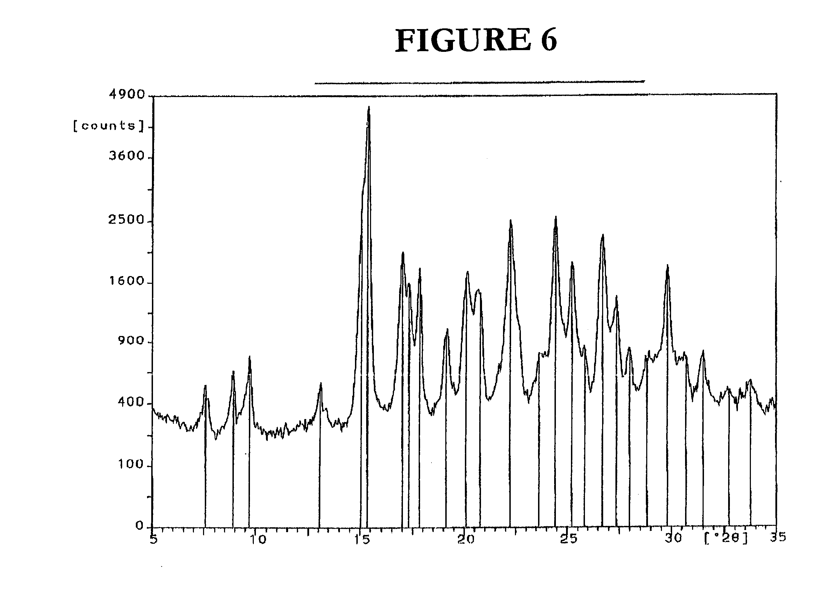

The three forms have a powder diffraction spectrum to X-rays characterized by the principal absorptions reported in Tables 1, 2 and 3 corresponding to Figures 4, 5 and 6, respectively (Radiation Cu Kα, Generator voltage 40 kV, Divergence Slit 1°, Receiving slit 0.2 mm, scan mode step start angle 5,000, End angle 35,000, time per step 2,000 sec):

**(Table 1)**

| FORM I | | |
|---|---|---|
| Angle (2θ) | d (Å) | Rel. Intens. (I/I₀) |
| 7.570 | 11.6687 | 2.4 |
| 8.580 | 10.2972 | 5.2 |
| 9.355 | 9.4458 | 8.1 |
| 14.005 | 6.3183 | 6.4 |
| 15.125 | 5.8529 | 41.4 |
| 16.005 | 5.5330 | 100.0 |
| 17.160 | 5.1631 | 10.0 |
| 18.625 | 4.7601 | 31.0 |
| 20.240 | 4.3838 | 6.8 |
| 21.000 | 4.2268 | 13.9 |
| 21.990 | 4.0387 | 32.9 |
| 22.785 | 3.8996 | 12.1 |
| 23.585 | 3.7691 | 30.0 |
| 25.055 | 3.5512 | 60.4 |
| 26.480 | 3.3632 | 18.0 |
| 28.425 | 3.1374 | 11.9 |
| 28.905 | 3.0863 | 8.6 |
| 30.430 | 2.9351 | 8.1 |
| 31.395 | 2.8470 | 6.7 |
| 32.145 | 2.7823 | 8.9 |
| 33.990 | 2.6353 | 9.3 |

**(Table 2)**

| FORM II | | |
|---|---|---|
| Angle (2θ) | d (Å) | Rel. Intens. (I/I₀) |
| 7.615 | 11.5998 | 7.4 |
| 8.985 | 9.8340 | 4.8 |
| 9.740 | 9.0733 | 9.3 |
| 13.635 | 6.4889 | 11.6 |
| 14.015 | 6.3138 | 7.1 |
| 15.320 | 5.7788 | 100.0 |
| 17.105 | 5.1796 | 43.8 |
| 17.910 | 4.9485 | 21.8 |
| 19.255 | 4.6058 | 16.7 |
| 20.330 | 4.3646 | 27.8 |
| 20.765 | 4.2741 | 21.7 |
| 22.285 | 3.9859 | 37.8 |
| 23.730 | 3.7464 | 14.1 |
| 24.610 | 3.6144 | 37.7 |
| 25.485 | 3.4922 | 27.0 |
| 27.030 | 3.2960 | 24.4 |
| 27.440 | 3.2477 | 17.0 |
| 28.135 | 3.1690 | 8.7 |
| 29.225 | 3.0533 | 12.7 |
| 29.905 | 2.9854 | 24.1 |
| 31.645 | 2.8251 | 11.5 |

**(Table 3)**

| FORM III | | |
|---|---|---|
| Angle (2θ) | d (Å) | Rel. Intens. (I/I₀) |
| 7.555 | 11.6918 | 6.2 |
| 8.895 | 9.9333 | 9.0 |
| 9.670 | 9.1388 | 12.1 |
| 13.050 | 6.7785 | 5.7 |
| 15.030 | 5.8896 | 55.2 |
| 15.345 | 5.7694 | 100.0 |
| 16.970 | 5.2205 | 40.3 |
| 17.300 | 5.1216 | 30.3 |
| 17.810 | 4.9761 | 34.7 |
| 19.105 | 4.6416 | 16.9 |
| 20.060 | 4.4227 | 33.0 |
| 20.745 | 4.2782 | 27.4 |
| 22.190 | 4.0028 | 51.0 |
| 24.400 | 3.6450 | 52.1 |
| 25.205 | 3.5304 | 36.7 |
| 25.830 | 3.4464 | 13.4 |
| 26.675 | 3.3391 | 46.0 |
| 27.360 | 3.2570 | 26.3 |
| 27.985 | 3.1857 | 13.2 |
| 29.795 | 2.9961 | 35.5 |
| 30.685 | 2.9112 | 11.4 |

The X-ray diffractions were carried out with a Philips PW3710 X-ray Diffractometer.

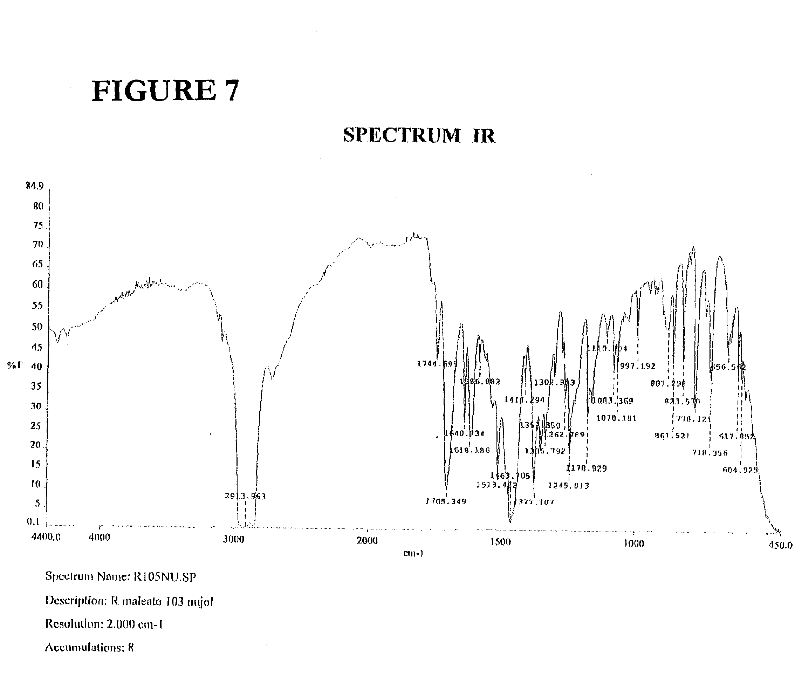

Form I exhibits with IR characteristic absorptions at the following wavelengths (Figure 7): 1744; 1618; 1262; 1178; 1083; 1070; 997, 823; 778 cm⁻¹.

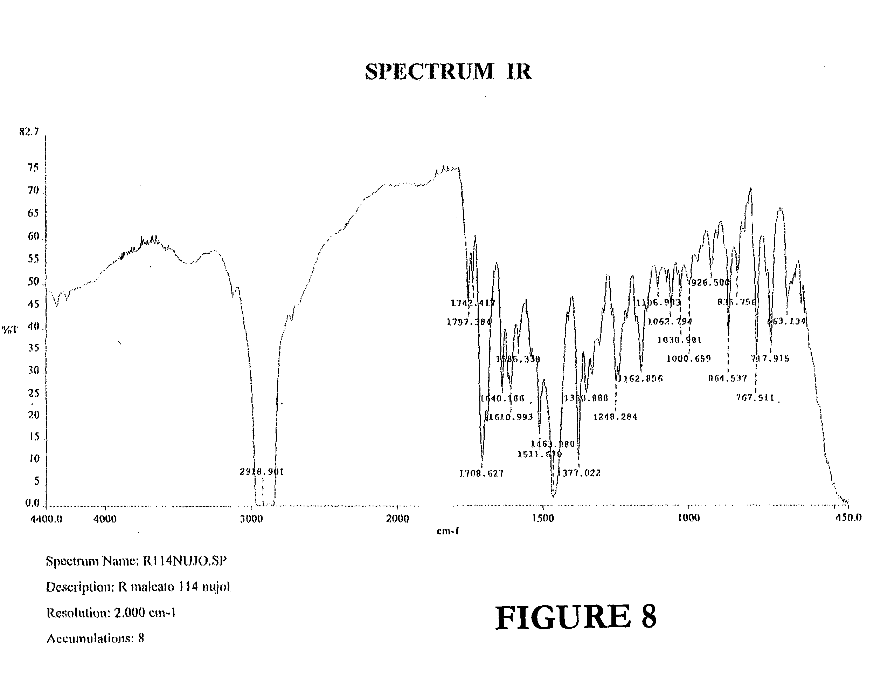

Form II exhibits with IR the following characteristic absorptions (Figure 8): 1757; 1610; 1162; 1062; 1030; 926; 835; 767 cm⁻¹.

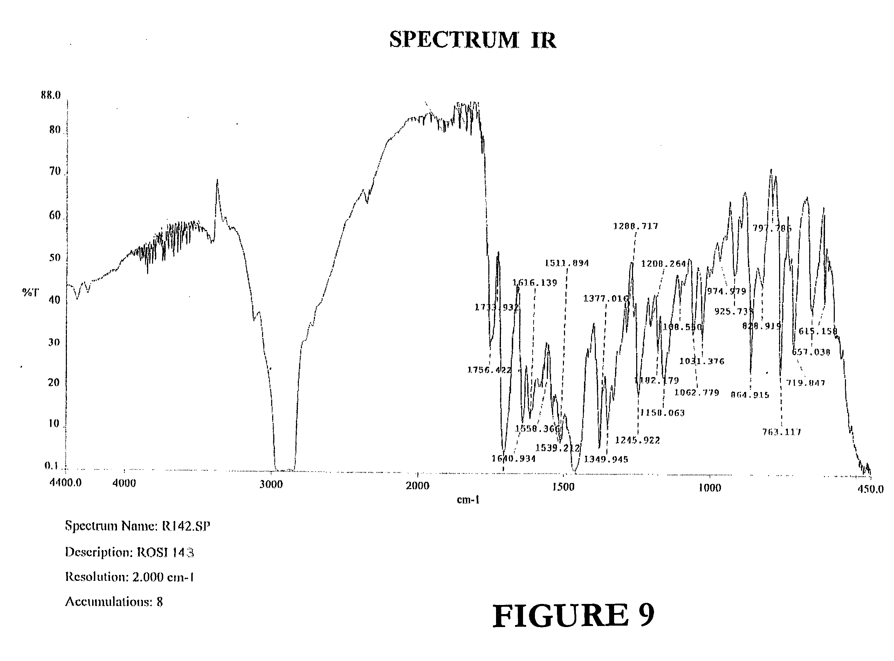

Form III, on the other hand, exhibits with IR the following characteristic absorptions (Figure 9): 1756; 1585; 1010; 921cm⁻¹.

The IR spectra were carried out with a Perkin Elmer 16 PC FT-IR spectrometer.

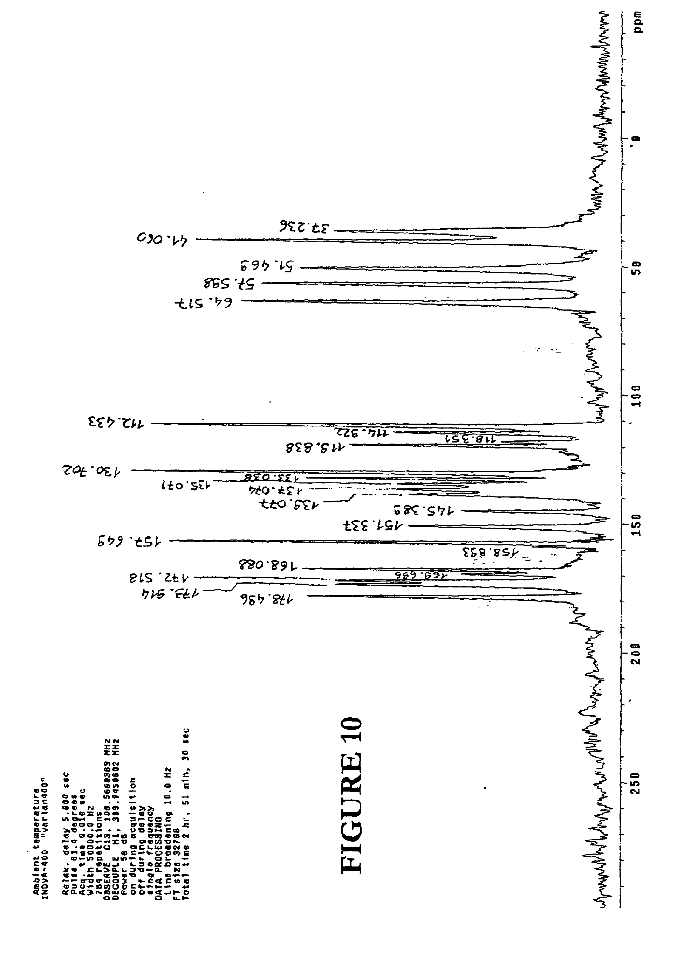

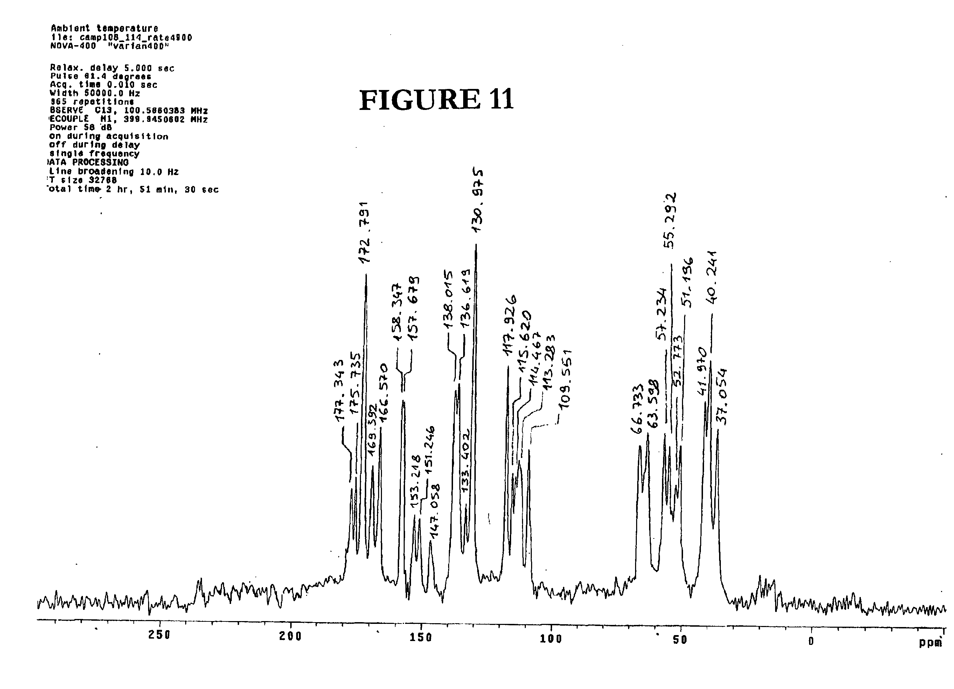

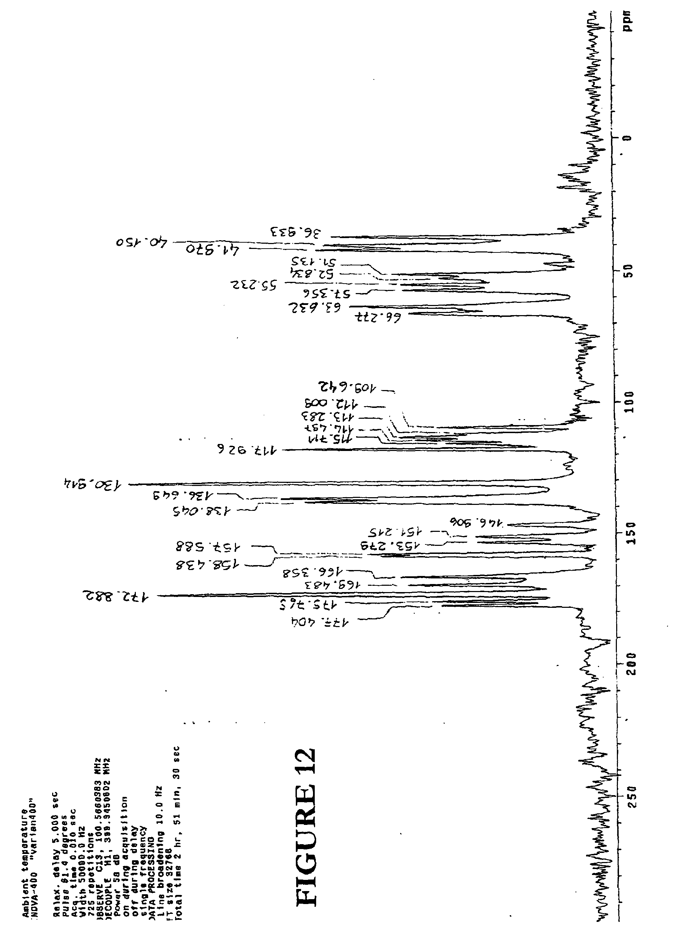

The solid-state ¹³C-NMR spectra of Forms I, II and III, obtained with a Varian 400 Unity Inova, are reported in figures 10, 11, 12 and in the following tables 4, 5 and 6, respectively:

### Chemical shifts (ppm):

**(Table )**

| FORM I | | | | |
|---|---|---|---|---|
| 178.5 | 168.1 | 145.4 | 133.0 | 51.5 |
| 173.9 | 158.9 | 139.1 | 130.7 | 41.1 |
| 172.5 | 157.6 | 137.1 | 64.5 | 37.2 |
| 169.7 | 151.3 | 135.1 | 57.6 | |

**(Table 5)**

| FORM II | | | | | | |
|---|---|---|---|---|---|---|
| 177.3 | 166.6 | 151.2 | 133.4 | 114.5 | 63.6 | 51.2 |
| 175.7 | 158.3 | 147.1 | 131.0 | 113.3 | 57.2 | 42.0 |
| 172.8 | 157.7 | 138.0 | 117.9 | 109.6 | 55.3 | 40.2 |
| 169.4 | 153.2 | 136.7 | 115.6 | 66.7 | 52.8 | 37.1 |

**(Table 6)**

| FORM III | | | | | | |
|---|---|---|---|---|---|---|
| 177.4 | 166.4 | 151.2 | 130.9 | 113.3 | 63.6 | 51.1 |
| 175.8 | 158.4 | 146.9 | 117.9 | 112.0 | 57.4 | 42.0 |
| 172.9 | 157.6 | 138.0 | 115.7 | 109.6 | 55.2 | 40.2 |
| 169.5 | 153.3 | 136.6 | 114.5 | 66.3 | 52.8 | 36.9 |

Rosiglitazone maleate may be obtained in the form of the single polymorph I by blending an approximately equimolar mixture of rosiglitazone base and maleic acid in a series of solvents and mixtures thereof, which comprises isopropanol, acetone, ethyl acetate, isopropyl acetate, THF, by heating the suspension to reflux temperature of the solvent, followed by cooling of the mixture to ambient temperature. In this way a crystalline suspension of the product is obtained which, when filtered, washed and desiccated under vacuum for 12 hours at 45-50°C provides rosiglitazone maleate form I as the single crystalline form, as confirmed by IR, XRD and DSC analyses.

Form II of rosiglitazone maleate, however, may be obtained in a pure form by treatment of the approximately equimolar mixture of rosiglitazone base and maleic acid in water under reflux, followed by cooling of the mixture to ambient temperature. The solid suspended in the mixture may be filtered, washed and desiccated under vacuum for about 10 to 14 hours, preferably 12 hours, at 45-50°C and consists exclusively of crystals of Form II of rosiglitazone maleate.

Alternatively Form II of rosiglitazone maleate may be prepared by mixing approximately equimolar quantities of rosiglitazone base and maleic acid in a water:ethanol mixture from 1.5 : 1 to 2.5 : 1 by volume, preferably 2 : 1, under reflux, followed by cooling of the mixture to ambient temperature. The solid suspended in the mixture may be filtered, washed and desiccated under vacuum for about 10 to 14 hours.

Form III of rosiglitazone maleate on the other hand may be obtained in a pure form by crystallization of rosiglitazone base and a double molar quantity of maleic acid in absolute ethanol or denatured ethanol. The mixture of the starting materials is brought to reflux, where a solution is obtained and it is then slowly cooled to room temperature; the crystalline solid thus formed is filtered, washed and dried and consists exclusively of crystals of Form III of rosiglitazone maleate.

The following experimental examples provide further clarification of the invention itself and in no way constitute any limitation thereof.

### EXAMPLE 1

### Synthesis of Rosiglitazone maleate Form I.

A 250 ml balloon flask equipped with mechanical stirring, coolant and thermometer, is charged with 10 g (28.0 mmoles) of rosiglitazone base, 3.25 g (28.0 mmoles) of maleic acid and 75 ml of isopropanol. The mixture is brought to reflux and maintained for 30' under such conditions. The mixture is then slowly cooled to ambient temperature and the product is filtered on a Buchner filter, washing twice with 10 ml of isopropanol. The filtered product is then desiccated for 12 hours at 45-50°C. 9.7 g of rosiglitazone maleate Form I (yield 73%) are obtained. The content of residual isopropanol in the product is 0.16% by weight.

### EXAMPLE 2

### Synthesis of Rosiglitazone maleate Form II.

A 500 ml balloon flask is charged with 20 g (56.0 mmoles) of rosiglitazone base and 6.50 g (56.0 mmoles) of maleic acid. To these solids are added 350 ml of water, and the mixture obtained is brought to reflux for 30'. The mixture is then slowly cooled to ambient temperature and the resultant solid is filtered on a Buchner filter, washing twice with 20 ml of water each time. A product is discharged which when desiccated under vacuum at 45-50°C for 12 hours weighs 19.9 g (yield 75%) and consists of rosiglitazone maleate Form II. The water content of the desiccated product is 0.3%.

### EXAMPLE 3

### Synthesis of rosiglitazone Form I.

Example 1 is repeated, using isopropyl acetate as solvent in place of the isopropanol. After desiccation, 9.5 g of rosiglitazone maleate Form I (yield 72%) are obtained.

### EXAMPLE 4

### Synthesis of Rosiglitazone maleate Form III.

A 500 ml balloon flask is charged with 15 g (42.0 mmoles) of rosiglitazone base and 9.70 g (84.0 mmoles) of maleic acid. To these solids are added 150 ml of ethanol, and the mixture obtained is brought to reflux for 30'. The mixture is then slowly cooled to ambient temperature and the resultant solid is filtered on a Buchner filter, washing twice with 20 ml of ethanol each time. A product is discharged which when desiccated under vacuum at 45-50°C for 12 hours weighs 14.9 g (yield 75%) and consists of rosiglitazone maleate Form III.

### EXAMPLE 5

### Synthesis of Rosiglitazone maleate Form II.

A 500 ml balloon flask equipped with reflux condenser and dropping funnel is charged with 20 g (56.0 mmoles) of rosiglitazone base and 330 ml of deionised water. In a becker are charged 6.50 g (56.0 mmoles) of maleic acid and 23 ml of deionised water, whereby a solution is formed. The solution obtained is then charged in the dropping funnel. The suspension of rosiglitazone base in water is heated to reflux and from the dropping funnel the solution of maleic acid is added in approximately 5'. The mixture is then slowly cooled to ambient temperature and the resultant solid is filtered on a Buchner filter, washing twice with 20 ml of water each time. A product is discharged which when desiccated under vacuum at 45-50°C for 12 hours weighs 20.5 g (yield 77%) and consists of rosiglitazone maleate Form II. The water content of the desiccated product is 0.4%.

### EXAMPLE 6

### Synthesis of Rosiglitazone maleate Form II.

A 500 ml balloon flask is charged with 20 g (56.0 mmoles) of rosiglitazone base and 6.50 g (56.0 mmoles) of maleic acid. To these solids are added 160 ml of water and 80 ml of absolute ethanol. The mixture obtained is brought to reflux for 30' to obtain a clear solution. The solution is filtered on a panel of celite and allowed to cool to ambient temperature. The resultant solid is filtered on a Buchner filter, washed twice with 20 ml of water each time. A product is discharged which when desiccated under vacuum at 45-50°C for 12 hours weighs 21.0 g (yield 79%) and consists of rosiglitazone maleate Form II. The water content of the desiccated product is 0.3%.

## Claims

1. Rosiglitazone maleate crystalline form I having a powder diffraction spectrum to X-rays with the following principal absorptions:
| Angle (2θ) | d (Å) | Rel. Intens. (I/I₀) |
|---|---|---|
| 7.570 | 11.6687 | 2.4 |
| 8.580 | 10.2972 | 5.2 |
| 9.355 | 9.4458 | 8.1 |
| 14.005 | 6.3183 | 6.4 |
| 15.125 | 5.8529 | 41.4 |
| 16.005 | 5.5330 | 100.0 |
| 17.160 | 5.1631 | 10.0 |
| 18.625 | 4.7601 | 31.0 |
| 20.240 | 4.3838 | 6.8 |
| 21.000 | 4.2268 | 13.9 |
| 21.990 | 4.0387 | 32.9 |
| 22.785 | 3.8996 | 12.1 |
| 23.585 | 3.7691 | 30.0 |
| 25.055 | 3.5512 | 60.4 |
| 26.480 | 3.3632 | 18.0 |
| 28.425 | 3.1374 | 11.9 |
| 28.905 | 3.0863 | 8.6 |
| 30.430 | 2.9351 | 8.1 |
| 31.395 | 2.8470 | 6.7 |
| 32.145 | 2.7823 | 8.9 |
| 33.990 | 2.6353 | 9.3 |

2. Rosiglitazone maleate crystalline form I having a powder diffraction spectrum to X-rays as shown in Figure 4.

3. Rosiglitazone maleate crystalline form I having a DSC graph as shown in Figure 1.

4. Rosiglitazone maleate crystalline form I having an IR spectrum as shown in Figure 7.

5. Rosiglitazone maleate crystalline form II having a powder diffraction spectrum to X-rays with the following principal absorptions:
| Angle (2θ) | d (Å) | Rel. Intens. (I/I₀) |
|---|---|---|
| 7.615 | 11.5998 | 7.4 |
| 8.985 | 9.8340 | 4.8 |
| 9.740 | 9.0733 | 9.3 |
| 13.635 | 6.4889 | 11.6 |
| 14.015 | 6.3138 | 7.1 |
| 15.320 | 5.7788 | 100.0 |
| 17.105 | 5.1796 | 43.8 |
| 17.910 | 4.9485 | 21.8 |
| 19.255 | 4.6058 | 16.7 |
| 20.330 | 4.3646 | 27.8 |
| 20.765 | 4.2741 | 21.7 |
| 22.285 | 3.9859 | 37.8 |
| 23.730 | 3.7464 | 14.1 |
| 24.610 | 3.6144 | 37.7 |
| 25.485 | 3.4922 | 27.0 |
| 27.030 | 3.2960 | 24.4 |
| 27.440 | 3.2477 | 17.0 |
| 28.135 | 3.1690 | 8.7 |
| 29.225 | 3.0533 | 12.7 |
| 29.905 | 2.9854 | 24.1 |
| 31.645 | 2.8251 | 11.5 |

6. Rosiglitazone maleate crystalline form II having a powder diffraction spectrum to X-rays as shown in Figure 5.

7. Rosiglitazone maleate crystalline form II having a DSC graph as shown in Figure 2.

8. Rosiglitazone maleate crystalline form II having an IR spectrum as shown in Figure 8.

9. Rosiglitazone maleate crystalline form III having a powder diffraction spectrum to X-rays with the following principal absorptions:
| Angle (2θ) | d (Å) | Rel. Intens. (I/I₀) |
|---|---|---|
| 7.555 | 11.6918 | 6.2 |
| 8.895 | 9.9333 | 9.0 |
| 9.670 | 9.1388 | 12.1 |
| 13.050 | 6.7785 | 5.7 |
| 15.030 | 5.8896 | 55.2 |
| 15.345 | 5.7694 | 100.0 |
| 16.970 | 5.2205 | 40.3 |
| 17.300 | 5.1216 | 30.3 |
| 17.810 | 4.9761 | 34.7 |
| 19.105 | 4.6416 | 16.9 |
| 20.060 | 4.4227 | 33.0 |
| 20.745 | 4.2782 | 27.4 |
| 22.190 | 4.0028 | 51.0 |
| 24.400 | 3.6450 | 52.1 |
| 25.205 | 3.5304 | 36.7 |
| 25.830 | 3.4464 | 13.4 |
| 26.675 | 3.3391 | 46.0 |
| 27.360 | 3.2570 | 26.3 |
| 27.985 | 3.1857 | 13.2 |
| 29.795 | 2.9961 | 35.5 |
| 30.685 | 2.9112 | 11.4 |

10. Rosiglitazone maleate crystalline form III having a powder diffraction spectrum to X-rays as shown in Figure 6.

11. Rosiglitazone maleate crystalline form III having a DSC graph as shown in Figure 3.

12. Rosiglitazone maleate crystalline form III having an IR spectrum as shown in Figure 9.

13. Pharmaceutical compositions containing rosiglitazone maleate crystalline form I according to claim 1 together with pharmaceutically acceptable excipients and/or adjuvants.

14. Pharmaceutical compositions containing rosiglitazone maleate crystalline form II according to claim 5 together with pharmaceutically acceptable excipients and/or adjuvants.

15. Pharmaceutical compositions containing rosiglitazone maleate crystalline form III according to claim 9 together with pharmaceutically acceptable excipients and/or adjuvants.

16. A process for the crystallization of rosiglitazone maleate form I **characterized in that** it comprises the following steps:
a. heating to reflux an approximately equimolar mixture of rosiglitazone base and maleic acid in a solvent selected from alcohols, esters and/or ethers;
b. cooling said mixture to ambient temperature;
c. filtration and washing of the product;
d. desiccation.

17. A process according to claim 16, **characterized in that** said alcohols and/or esters are selected from isopropanol, ethyl acetate, isopropyl acetate and/or THF.

18. A process for the crystallization of rosiglitazone maleate form II, **characterized in that** it comprises the following steps:
a. heating to reflux an approximately equimolar mixture of rosiglitazone base and maleic acid in water;
b. cooling said mixture to ambient temperature;
c. filtration and washing of the product;
d. desiccation.

19. A process for the crystallization of rosiglitazone maleate form II, **characterized in that** it comprises the following steps:
a. heating to reflux an approximately equimolar mixture of rosiglitazone base and maleic acid in a water:ethanol mixture from 1.5 : 1 to 2.5 : 1 by volume;
b. cooling said mixture to ambient temperature;
c. filtration and washing of the product;
d. desiccation.

20. A process for the crystallization of rosiglitazone maleate form III **characterized in that** it comprises the following steps:
a. heating to reflux a mixture approximately containing rosiglitazone base and a double molar quantity of maleic acid in absolute ethanol and/or denatured ethanol;
b. cooling said mixture to ambient temperature;
c. filtration and washing of the product;
d. desiccation.

21. A process according to claims 16 to 20, **characterized in that** said mixture is maintained under reflux for a time ranging between about 20 and 40 minutes.
